(19) Europäisches Patentamt
European Patent Office
Office européen des brevets

(11) **EP 3 774 944 B1**

(12) **EUROPEAN PATENT SPECIFICATION**

(45) Date of publication and mention of the grant of the patent:
**13.04.2022 Bulletin 2022/15**

(21) Application number: **19724525.1**

(22) Date of filing: **21.05.2019**

(51) International Patent Classification (IPC):
***C08F 212/14*** (2006.01) ***C08F 220/28*** (2006.01)
***C08L 33/06*** (2006.01) ***C09D 133/06*** (2006.01)
***C09B 69/10*** (2006.01) ***A61K 6/65*** (2020.01)
***A61Q 11/00*** (2006.01) ***A61C 19/06*** (2006.01)
***A61K 8/81*** (2006.01)

(52) Cooperative Patent Classification (CPC):
(C-Sets available)
**C09B 69/103; A61C 19/066; A61K 6/65; A61K 8/8152; A61Q 11/00; C08F 212/21; C08L 33/06; C09D 133/06;** A61K 2800/434; A61K 2800/88; C08F 12/21; C08F 12/22; C08F 12/24; C08F 220/286 (Cont.)

(86) International application number:
**PCT/EP2019/063074**

(87) International publication number:
**WO 2020/001864 (02.01.2020 Gazette 2020/01)**

(54) **A NOVEL POLYMER, A COMPOSITION, A METHOD AND A KIT FOR WHITENING TEETH**

NEUARTIGES POLYMER, ZUSAMMENSETZUNG, VERFAHREN UND KIT ZUM BLEICHEN VON ZÄHNEN

NOUVEAU POLYMÈRE, COMPOSITION, PROCÉDÉ ET KIT DE BLANCHIMENT DES DENTS

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **26.06.2018 PCT/CN2018/092902**
**26.07.2018 EP 18185765**

(43) Date of publication of application:
**17.02.2021 Bulletin 2021/07**

(73) Proprietors:
- **Unilever Global IP Limited**
  **Wirral, Merseyside CH62 4ZD (GB)**
  Designated Contracting States:
  **CY DE GB IE IT MT**
- **Unilever IP Holdings B.V.**
  **3013 AL Rotterdam (NL)**
  Designated Contracting States:
  **AL AT BE BG CH CZ DK EE ES FI FR GR HR HU IS LI LT LU LV MC MK NL NO PL PT RO RS SE SI SK SM TR**

(72) Inventors:
- **GHATLIA, Naresh, Dhirajlal**
  **Bangalore 560 066 (IN)**
- **LAHORKAR, Praful, Gulab, Rao**
  **Bangalore 560 066 (IN)**
- **LIU, Shiyong**
  **Hefei, Anhui 230026 (CN)**
- **YANG, Xiaoxia**
  **Development Zone 200335 (CN)**
- **YAO, Chenzhi**
  **Hefei, 230026 (CN)**
- **VAIDYA, Ashish, Anant**
  **Bangalore 560 066 (IN)**

(74) Representative: **Tansley, Sally Elizabeth et al**
**Unilever N.V.**
**Unilever Patent Group**
**Bronland 14**
**6708 WH Wageningen (NL)**

(56) References cited:
**WO-A1-2016/031574 WO-A2-2010/149534**
**GB-A- 1 400 892**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- **MUNISH CHANANA ET AL: "Fabrication of Colloidal Stable, Thermosensitive, and Biocompatible Magnetite Nanoparticles and Study of Their Reversible Agglomeration in Aqueous Milieu", CHEMISTRY OF MATERIALS, vol. 21, no. 9, 13 April 2009 (2009-04-13) , pages 1906-1914, XP055406181, ISSN: 0897-4756, DOI: 10.1021/cm900126r**


(52) Cooperative Patent Classification (CPC): (Cont.)

C-Sets
**C08F 212/21;**
**C08L 33/06;**
**C09D 133/06;**
C08F 220/286

## Description

### Field of the Invention

The present invention relates to a novel polymer for use in an oral care composition for whitening teeth. More specifically, the invention relates to such a composition and a kit that contains the composition and a source of light.

### Background of the Invention

The colour of our teeth is influenced by a combination of their intrinsic colour and the presence of any extrinsic stains that may form thereon. Staining is linked to the adsorption of materials into the pellicle on the surface of enamel. Factors that influence extrinsic stain formation include poor brushing techniques, smoking, dietary intake of coloured foods (e.g. red wine), age and the use of certain cationic agents such as chlorhexidine or metallic salts of tin and iron. Consumers have strong desire for whiter teeth and some individuals are dissatisfied with the inherent colour of their teeth or with the stains. This desire for whiter teeth has given rise to a growing trend of whitening products which range from toothpastes to mouthwashes and chewing gums.

Toothpastes meant for whitening rely on an optimization of abrasive and chemical components for removal of stains. During brushing, these abrasive particles get temporarily trapped between the toothbrush and the stained surface (of teeth) to abrade away the stains. Chemical components may also be used, usually in conjunction with abrasive particles, and these include calcium chelators, polymers, surfactants, enzymes and oxidising agents.

One of the more prevalent methods involves the use of dentifrices which contain a bleaching agent such as hydrogen peroxide with one or more other bleaching agents as disclosed in US2002122776 A and WO0018366 A1 (Both Unilever).

EP 1935395 A1 (Unilever) describes a novel optical approach to tooth whitening in which the toothpaste contains a blue pigment (in particular Blue Covarine) which gets deposited on the surface of teeth. Here it is able to alter the optical effects of surface which leads to a perception of whiter teeth. Deposition is aided by GANTREZ® type of polymers.

US2009238778 AA (MCNEIL PPC) discloses tooth whitening compositions that are capable of being activated by light energy. The compositions contain riboflavin or a derivative of riboflavin, a bleaching agent and a water-soluble liquid phase.

The present invention is directed to a novel polymer for inclusion in an oral care composition that provides enhanced tooth whitening over that provided by compositions disclosed hitherto while not depending on bleaching agents. The present inventors have tried to achieve this by tagging a photosensitive dye on to a polymer and then activating the dye by shining light on to the teeth coated with said polymer. They found that certain dyes of the xanthene class when bonded to polymers of a specific class are capable of whitening teeth better than known compositions when light is incident on the teeth coated with said polymers.

WO2010149534 discloses an oral care composition comprising a dye polymer for modifying the colour of teeth, which primarily comprises anthraquinone moieties. They work through direct action of the dye on the teeth and do not provide enhanced whiteness when light is shone on it.

It is thus an object of the present invention to provide for a novel polymeric dye that could be incorporated in an oral care composition to provide enhanced tooth whitening.

It is another object of the present invention to provide for an oral care composition comprising the novel polymeric dye that exhibits enhanced tooth whitening when light is shone on the teeth coated with said composition.

### Summary of the Invention

The first aspect of the present invention relates to a polymer having the structure

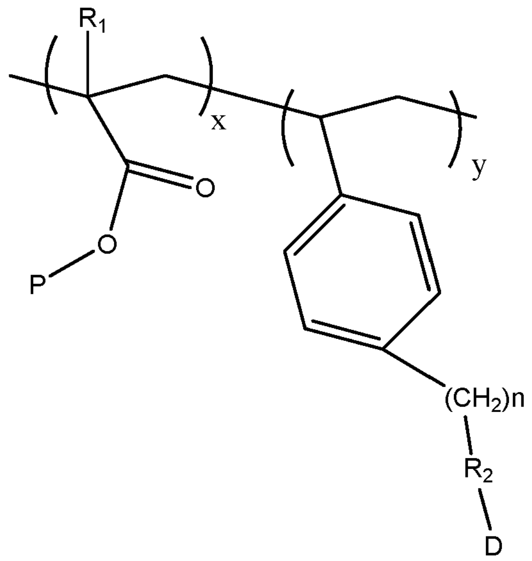

Where

$R_1$ is $C_n H_{2n+1}$
$R_2$ is a diheterofunctional group
n has a value from 1 to 10
x has a value from 1 to 10,000
y has a value from 1 to 10,000
P is a polyether chain; and
D is a Xanthene dye
selected from at least one of Acid Red 52, Acid Red 289, Gallein, Bromopyrogallol Red, 2',7'-Dichlorofluorescein Sodium Salt, 9-(4'-Dimethylaminophenyl)-2,6,7-trihydroxyfluorone Sulfate Hydrate, Dibromofluorescein, Uranine K, Fluorescein, Uranine, Pyrogallol Red, Sulfonfluorescein, Tetrabromofluorescein Potassium Salt, Acid Red 91 ( Eosin Bluish), Acid Red 94 (Rose Bengal), Acid Red 87( Eosin), Acid Red 92 (Phloxine B ), 3,4,5,6-Tetrachlorofluorescein, Tetraiodofluorescein (Erythrosin B or Erythrosine B) and Phenylfluorone.

It is particularly preferred that the xanthene dye is one of Rose Bengal, Eosin, Phloxine B, Fluorescin, or Erythrosine.

The second aspect relates to an oral care composition comprising a polymer of the first aspect and a cosmetically acceptable carrier.

According to a third aspect of the present invention there is provided a process to prepare the polymer of the first aspect of the present invention comprising the steps of first synthesizing the dye monomer and then synthesizing the dye polymer. The generalized process comprises the steps of:

(a) synthesizing the dye monomer by refluxing a mixture of the dye, chloromethylstyrene, potassium carbonate, and dimethyl formamide for six to ten hours; washing with salt solution, drying, filtering and concentrating to prepare the dye monomer; and
(b) synthesizing the dye polymer by repeated cycles of reacting OEGMA (oligo(ethylene glycol) methyl ether acrylate), dye monomer, AIBN (Azobisisobutyronitrile) and dioxane under an inert atmosphere for 1-10 hours at a temperature in the range of 20 to 70 °C, and precipitating in excess ether; followed by drying under vacuum at low temperature in the range of 20 to 40°C.

Another aspect of the present invention relates to a kit-of-parts comprising:

(i) An oral care composition of the second aspect;
(ii) a source of actinic/ Visible/LED light;
(iii) optionally, an applicator for applying said composition to teeth; and,
(iv) further optionally, a set of instructions for use of said kit-of-parts.

Yet another aspect of the present invention relates to a method of whitening teeth comprising applying the oral

care composition of the second aspect on to a tooth surface and exposing said composition, so applied, to a source of actinic/ Visible/LED.

Yet another aspect of the present invention relates to use of the oral care composition of the second aspect for whitening teeth.

Yet another aspect of the present invention relates to a packaged product comprising the oral care composition of the second aspect of the present invention.

## Detailed Description of the Invention

Any feature of one aspect of the present invention may be utilized in any other aspect of the invention. The word "comprising" is intended to mean "including" but not necessarily "consisting of' or "composed of." In other words, the listed steps or options need not be exhaustive. Except in the operating and comparative examples, or where otherwise explicitly indicated, all numbers in this description indicating amounts of material or conditions of reaction, physical properties of materials and/or use are to be understood as modified by the word "about". Numerical ranges expressed in the format "from x to y" are understood to include x and y. When for a specific feature multiple preferred ranges are described in the format "from x to y", it is understood that all ranges combining the different endpoints are also contemplated.

All references to the terms wt% or % by weight shall be construed as referring to the % of the concerned ingredient by weight of the composition, unless indicated to the contrary.

The term "actinic light" is intended to mean light energy emitted from a specific light source (e.g. visible, UV, lamp, LED, or laser) and capable of being absorbed by matter (e.g. the chromophore or photoactivator defined below). It is preferred that the actinic light is visible light.

The term "photosensitizer" refers to any agent capable of absorbing the actinic light. The photosensitive agent undergoes photoexcitation and transfers its energy. This may be in the form of emitted light (e.g. fluorescence) and/or energy transferred to molecules. This energy may initiate chemical reactions. Photosensitizers may enhance and/or accelerate the dispersion of light energy, or otherwise enhance and/or activate the decomposition of an oxidizing agent.

When referring to the pH of the aqueous gel composition, the pH is measured when 1 part by weight of the composition is uniformly dispersed and/or dissolved in 20 parts by weight pure water at 25 °C. In particular, the pH may be measured by manually mixing 1 g composition with 20 ml water for 30 seconds, then immediately testing the pH with indicator paper or a pH meter.

Viscosity of the composition is the value observed and recorded at room temperature (25 °C) with a Brookfield Viscometer, Spindle No. 4 and at a speed of 5 rpm. Values are quoted in centipoises (cP = mPa.s) unless otherwise specified.

Stained teeth could become troublesome, whether the cause for it is intrinsic or an extrinsic factor or agent like certain beverages chlorhexidine. The accumulation of stains on teeth poses an aesthetic problem for some individuals. Stains are usually extrinsic in nature and generally represent discoloration of the pellicle and/or plaque. The exact mechanisms involved in stain formation may not be fully understood. It is suggested that pigments produced by chromagenic bacteria, colored products from the chemical transformation of pellicle components and adsorption/retention of dietary constituents contribute to extrinsic stains. The dietary factors which appear to contribute heavily towards staining include coffee, tea, and red wines, as well as smoking. In addition to chlorhexidine, staining is promoted by other cationic antimicrobial agents such as hexetidine or quaternary ammonium compounds.

The efficacy of such compositions and kits can be measured and assessed by a term known as Whiteness Index Observed (WIO). The index proposed by Luo et al (known as WIO) has the same functional form as some of the previous whiteness indices but the parameters are different and are optimized specifically for the evaluation of whiteness in teeth. It is regarded as most appropriate for assessing changes in teeth whiteness.

Luo et al. proposed a modified version of the CIE whiteness index in Garcia JA, Rubino M, Romero J, Hita E. Measuring The Whiteness Of Human Teeth. Color Research and Application 1993; 18: 349 - 52. It is based directly on observers scoring of the Vita@ 3D Shade Guide and is defined as:

$$WIO = Y + 1075.012 (x_p - x) + 145.516 (y_p - y)$$

### The Polymeric dye of the Invention

The polymeric dye of the present invention has the general structure as follows:

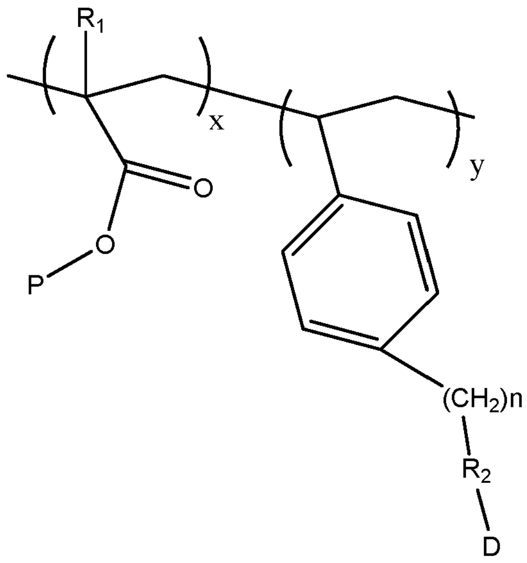

Where

$R_1$ is $C_n\ H_{2n+1}$
$R_2$ is a diheterofunctional group
n has a value from 1 to 10
x has a value from 1 to 10,000
y has a value from 1 to 10,000
P is a polyether chain; and
D is a Xanthene dye selected from at least one of Acid Red 52, Acid Red 289, Gallein, Bromopyrogallol Red, 2',7'-Dichlorofluorescein Sodium Salt, 9-(4'-Dimethylaminophenyl)-2,6,7-trihydroxyfluorone Sulfate Hydrate, Dibromofluorescein, Uranine K, Fluorescein, Uranine, Pyrogallol Red, Sulfonfluorescein, Tetrabromofluorescein Potassium Salt, Acid Red 91 ( Eosin Bluish), Acid Red 94 (Rose Bengal), Acid Red 87( Eosin), Acid Red 92 (Phloxine B ), 3,4,5,6-Tetrachlorofluorescein, Tetraiodofluorescein (Erythrosin B or Erythrosine B) and Phenylfluorone.

In the above structure, n preferably has a value from 1 to 5. The most preferred value of n is 1. In such a case, $R_1$ is most preferably $CH_3$. $R_2$ is a diheterofunctional group selected from -O-,or COO- more preferably -O-.

Although the polymer chain can be very long with x having a value from 1 to 10,000 and y having a value from 1 to 10,000, x preferably has a value from 1 to 500, more preferably from 5 to 100. Similarly y preferably has a value from 1 to 500, more preferably from 5 to 100.

The group P in the above general structure is preferably a polyethylene glycol chain having the structure

P = Polyethylene glycol chain

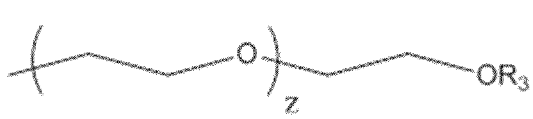

R3 = H or Phosphate or -CnH2n+l

In the above polyethylene glycol group it is preferred that n has a value from 1 to 10. In the above polyethylene glycol group it is preferred that z has a value from 1 to 10.

In the dye polymer of the first aspect of the present invention, the dye group D is one that comprises a Xanthene group.

One of the general structures of xanthene class of dye is as follows:

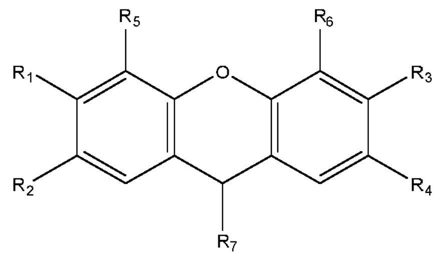

Where $R_1$, $R_2$, $R_3$, $R_4$, $R_5$ and $R_6$ are functional groups such as -OH, H, Br, I, Cl, amines, ketones, $O^-M^+$( where M is metal ion) and $R_7$ is organic moiety / derivative such as substituted aromatic or benzofuran.

As per a preferred aspect of the invention the photosensitizer is selected from a xanthene class of dye selected from one of Acid Red 52, Acid Red 289, Gallein, Bromopyrogallol Red, 2',7'-Dichlorofluorescein Sodium Salt, 9-(4'-Dimethylaminophenyl)-2,6,7-trihydroxyfluorone Sulfate Hydrate, Dibromofluorescein, Uranine K, Fluorescein, Uranine, Pyrogallol Red, Sulfonfluorescein, Tetrabromofluorescein Potassium Salt, Acid Red 91 ( Eosin Bluish), Acid Red 94 (Rose Bengal), Acid Red 87( Eosin), Acid Red 92 (Phloxine B ), 3,4,5,6-Tetrachlorofluorescein, Tetraiodofluorescein (Erythrosin B or Erythrosine B) and Phenylfluorone.

However, it is particularly preferred that the photosensitizer is at least one of Rose Bengal, Eosin, Phloxine B, Fluorescein or Erythrosine.

General structure of a preferred form of xanthene dye is shown below:

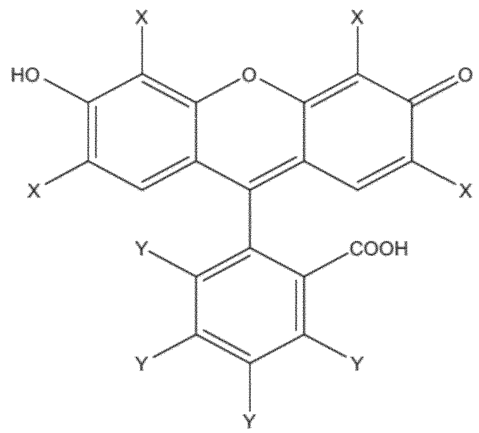

Referring to the chemical structure indicated above, in Fluorescein the X=H and Y=H; in Eosin the X-Br and Y=H; in Erythrosine the X=I and Y=H and in Rose Bengal the X=I and Y=Cl.

An especially preferred aspect of the present invention relates to polymer of the first aspect wherein D is an eosin group having the chemical structure:

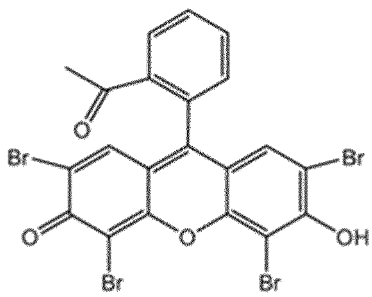

Eosin

When Eosin is the dye, the monomer reacts and binds to the COOH group of Eosin.

The composition of the invention is meant for whitening teeth which implies that it is useful for maintaining the whiteness of teeth. Alternatively, the composition of the invention is useful for removing or lightening, at least partly, some of the common stains such as tea or coffee stain.

According to a second aspect of the invention there is provided an oral care composition comprising a polymer as of the first aspect and a cosmetically acceptable carrier.

The oral care composition preferably comprises 0.1 to 10, more preferably 0.1 to 5 wt% of the polymer, by weight of the composition.

The pH of the aqueous gel composition of the invention is preferably from 3 to 11, and more preferably from 6 to 8.

The composition according to the invention may optionally include further ingredients to enhance performance and/or consumer acceptability, which are briefly discussed hereinafter.

Suitable surfactants include anionic surfactants, such as the sodium, magnesium, ammonium or ethanolamine salts of C8 to C18 alkyl sulphates (for example sodium lauryl sulphate), C8 to C18 alkyl sulphosuccinates (for example dioctyl sodium sulphosuccinate), C8 to C18 alkyl sulphoacetates (such as sodium lauryl sulphoacetate), C8 to C18 alkyl sarcosinates (such as sodium lauryl sarcosinate), C8 to C18 alkyl phosphates (which can optionally comprise up to 10 ethylene oxide and/or propylene oxide units) and sulphated monoglycerides. Other suitable surfactants include nonionic surfactants, such as optionally polyethoxylated fatty acid sorbitan esters, ethoxylated fatty acids, esters of polyethylene glycol, ethoxylates of fatty acid monoglycerides and diglycerides, and ethylene oxide/propylene oxide block polymers. Other suitable surfactants include amphoteric surfactants, such as betaines or sulphobetaines. Mixtures of any of the above described materials may also be used.

The level of surfactant may generally range from 0.2 to 15% by total weight surfactant based on the total weight of the composition.

A gelling agent is preferably included along with the polymer to obtain the desired viscosity. Preferred gelling agents are a copolymer of ammonium acryloyldimethyltaurate and vinylpyrrolidone, a modified, gelatin or polyvinylalcohol or combinations thereof. Gelling agents may be included in 0.1 to 10%, more preferably 0.1 to 2% by weight of the composition.

Also suitable are fluoride sources such as sodium fluoride, stannous fluoride, sodium monofluorophosphate, zinc ammonium fluoride, tin ammonium fluoride, calcium fluoride, cobalt ammonium fluoride and mixtures thereof; plant-derivable antioxidants such as flavonoid, catechin, polyphenol, and tannin compounds and mixtures thereof; antioxidant vitamins such as tocopherols and/or derivatives thereof, ascorbic acid and/or derivatives thereof and mixtures thereof. Further optional ingredients customary in the art such as buffers, flavouring agents, sweetening agents, colouring agents, opacifying agents and preservatives, may also be included.

According to a third aspect of the present invention there is provided a process to prepare the polymer of the first aspect of the present invention comprising the steps of first synthesizing the dye monomer and then synthesizing the polymer. The generalized process comprises the steps of:

(a) synthesizing the dye monomer by refluxing a mixture of the dye, chloromethylstyrene, potassium carbonate, and dimethyl formamide for six to ten hours; washing with salt solution, drying, filtering and concentrating to prepare the dye monomer; and

(b) synthesizing the dye polymer by repeated cycles of reacting OEGMA (oligo(ethylene glycol) methyl ether acrylate), dye monomer, AIBN (Azobisisobutyronitrile) and dioxane under an inert atmosphere for 1-10 hours at a temperature in the range of 20 to 70 °C, and precipitating in excess ether; followed by drying under vacuum at low temperature in the range of 20 to 40°C.

More details on the general process includes two parts (i) To synthesize the dye monomer and (ii) to synthesize the polymer

Synthesis of dye monomer

Dye, chloromethylstyrene, potassium carbonate, and dimethyl formamide were charged into a reaction flask, the mixture was reflux for 6-10 h. After evaporating all the solvents, the residues were dissolved in methylene dichloride ($CH_2Cl_2$), successively washed with saturated aqueous sodium chloride and water, dried over anhydrous magnesium sulphate, filtered, and then concentrated on a rotary evaporator.

Synthesis of dye polymer

*OEGMA* (*oligo(ethylene glycol) methyl ether acrylate),* dye monomer, AIBN (Azobisisobutyronitrile) and dioxane were charged into a reaction flask. The mixture was degassed by three freeze-pump-thaw cycles and backfilled with nitrogen. After stirring for 5-10 h at 70°C, the reaction tube was quenched into liquid $N_2$, opened and exposed to air, and precipitated into an excess of ether. The above dissolution-precipitation cycle was repeated for three times. The final product was dried in a vacuum oven overnight at room temperature, yielding a red solid.

The composition of the invention is preferably a gel. It is preferred that viscosity of the gel is from 200 to 100,000

cps, more preferably 2000 to 50,000 cps.

The kit-of-parts of the Invention

In accordance with a further aspect, the invention relates to a kit-of-parts comprising:

(i) an oral care composition of the second aspect;
(ii) a source of actinic/ Visible/LED light;
(iii) optionally, an applicator for applying said composition to teeth; and,
(iv) further optionally, a set of instructions for use of said kit-of-parts.

The composition in accordance with the invention needs to be used in conjunction with a suitable source of actinic light.

Any suitable source of actinic light can be used so long as the light is able to activate the photosensitizer belonging to the xanthene group. The primary characteristic of suitable sources of actinic light will be that they emit light in a wavelength (or wavelengths) appropriate for photosensitization of the photosensitizer present in the composition.

Preferably the source of actinic light is halogen bulb, LED or plasma arc lamp, or laser such as an argon laser, potassium-titanyl phosphate (KTP) laser, LED photocuring device. The radiation is preferably of the visible range but it may be UV or IR. It is preferred that the actinic light has a wavelength in the range from about 400 nm to about 700 nm. In another embodiment, the actinic light has wavelength in the range from about 420 nm to about 560 nm, more preferably 435 nm to about 550 nm.

The applicator is preferably the one which is disclosed in US 20030194383 A (Unilever). This applicator or tray comprises a U-shaped dental tray fittable over a person's teeth which permits contact of the composition of the invention with all possible surfaces surrounding the teeth. Alternatively, the applicator is any other suitable applicator known in the art.

The set of instructions is preferably in the form of a pamphlet containing useful information about the contents and step-by-step instructions for use of the compositions of the invention.

Without wishing to be bound by theory the inventors believe that with the polymer of the first aspect of the present invention where the photosensitizer is bound to the polymer there is better deposition of the polymer on the teeth and thereby the photosensitizer moiety; so that when it is exposed to preferably visible white light, it produces reactive oxygen species which in turn interact with the stains chromophores present on teeth breaking it down to small colorless molecules which get washed off thereby removing/lightening the stains. The result is whiter teeth.

The device

The device for whitening teeth comprises:

(i) an applicator for applying to the teeth an oral care composition of the second aspect; and,
(ii) a source of actinic light integral with said device.

As an example, reference may be made to the fittable tray disclosed earlier, with provision for flashing visible light. In such a case it is preferred that the device comprises a switch or button to switch on and off the light source. The device preferably also includes a battery for supplying energy to the source of light. In such a case it is preferred that the device is easy to use.

The packaged product of the Invention

In accordance with another aspect is disclosed a packaged product comprising the oral care composition of the invention. The composition is preferably packaged in a collapsible tube such that it becomes easy to dispense a small amount without much dexterity. Alternatively, the composition is packaged in another type of container, such as a bottle or a jar, preferably with suitable means for dispensing a known quantity of the composition. The pack may be further packed in a suitable secondary package like a carton or a box, preferably with useful information about the contents and instructions for use.

The method of the Invention

In accordance with another aspect is disclosed a method of whitening teeth comprising:

(i) applying the oral care composition of the second aspect on to a tooth surface; and
(ii) exposing said composition, so applied, to a source of actinic/ Visible/LED light.

Preferably the composition is applied using the applicator as disclosed earlier. After application, the teeth are exposed to visible light preferably for 5 to 45 minutes, more preferably for less than 10 minutes. Alternatively, the exposure lasts less than 5 minutes, such as 4, 3, 2, or 1 minute. The application may be repeated as often as desired, e.g. 2 to 3 times a day and 3 to 4 days in a fortnight. The method preferably results in at least one unit of shade difference on the Vita® Shade card, preferably at least 2 units and more preferably at least 3-5 units. The method is relatively free of post-treatment sensitivity. In some other cases, there is minimal or acceptable level of post-treatment sensitivity. The whitening effect may diminish over time following a treatment. In that case, repeat of the treatment is advisable.

The method for whitening the teeth may be performed in a dentist's office or clinic under ordinary or supervised conditions. Alternatively, and more preferably the method is performed by an informed user at home or in other words, without any medical supervision when the method is non-therapeutic.

The composition and method of the invention is useful to whiten teeth. This means that the composition is useful to preserve the white appearance of teeth or to restore the appearance periodically. Alternatively, the composition and the method is useful to lighten the teeth which are discoloured due to extrinsic or intrinsic stains (e.g., tobacco, coffee, tea and/or food or wine stains), fluorosis, developmental disturbances, bacteria, genetics, tetracycline antibiotics, trauma, blood decomposition, and pigments present during development of teeth.

In one aspect the method is non-therapeutic. Preferably such a method is a cosmetic method. A therapeutic treatment starts from a pathological state, whereas a non-therapeutic treatment starts from a normal, healthy state. Application of such a method to a person is to improve his bodily appearance (cosmetic treatment).

In another aspect the method is therapeutic in nature. Treatment by therapy is defined as any treatment which is designed to cure, alleviate, remove or lessen the symptoms of, or prevent or reduce the possibility of contracting any disorder or malfunction of the human body, in this case it is teeth.

Use in accordance with the Invention

In accordance with yet another aspect is disclosed an oral care composition of the second aspect for whitening teeth.

In one aspect the use is for non-therapeutic purpose. Alternatively, the use is for therapeutic purpose. The part of the description related to the method of the invention is also applicable *mutatis mutandis* to this aspect of the invention.

The following examples will more fully illustrate the embodiments of this invention. All parts, percentages and proportions referred to herein and in the appended claims are by weight unless otherwise illustrated.

## Examples

*Synthesis of Eosin-based Polymerizable Dye EoS.*

Eosin Y (647 mg, 1 mmol), chloromethylstyrene (152 mg, 1 mmol), potassium carbonate (275 mg, 2 mmol), and dimethyl formamide (DMF) (5 ml) were charged into a reaction flask, the mixture was reflux for 6 h. After evaporating all the solvents, the residues were dissolved in methylene dichloride ($CH_2Cl_2$), successively washed with saturated aqueous sodium chlloride and water, dried over anhydrous magnesium sulphate, filtered, and then concentrated on a rotary evaporator. The crude product was subjected to further purification by silica gel column chromatography using $CH_2Cl_2$/methanol (98/2 v/v) as the eluent, affording 4-vinylbenzyl 2-(2,4,5,7-tetrabromo-6-hydroxy-3-oxo-3H-xanthen-9-yl) benzoate (EoS) as a residual solid (540 mg, 67 % yield).$^1$H NMR (DMSO-d6, $\delta$, ppm, TMS): 8.22 (d, 1H, aromatic protons), 7.92 (m, 2H, aromatic protons), 7.42 (d, 1H, aromatic protons), 7.25 (d, 2H, aromatic protons), 6.95 (s, 2H, aromatic protons), 6.82 (d, 2H, aromatic protons), 6.62 (q, 1H, -C*H*=$CH_2$), 5.76 (d, 1H, -CH=C*H*H), 5.28 (d, 1H, -CH=CH*H*), 4.96 (s, 2H, COO-C$H_2$-).

Example - 1: Synthesis of Poly(OEGMA-co-EoS). *OEGMA- oligo(ethylene glycol) methyl ether acrylate*

*OEGMA* (9.5 g, 20 mmol), EoS (0.015 g, 0.2 mmol), AIBN Azobisisobutyronitrile (0.008 g, 0.05 mmol), and dioxane (19 mL) were charged into a reaction flask. The mixture was degassed by three freeze-pump-thaw cycles and backfilled with nitrogen. After stirring for 5 h at 70°C, the reaction tube was quenched into liquid $N_2$, opened and exposed to air, and precipitated into an excess of ether. The above dissolution-precipitation cycle was repeated for three times. The final product was dried in a vacuum oven overnight at room temperature, yielding a red solid (6.6 g, yield: 69 %). The molecular weight and molecular weight distribution of Poly(OEGMA-co-EoS) were determined by GPC using DMF as the eluent, revealing an $M_n$ of 22,300 and $M_w/M_n$ of 1.84.

The polymer as prepared above (with eosin content equivalent to 0.02% Eosin) was formulated as a gel with 1% of Aristoflex® AVC.

Example 2: A gel was formulated with 0.02% Eosin in 1% of Aristoflex® AVC

Example - 3: A control gel with 1% of Aristoflex® AVC with no Eosin was prepared.

Photobleach Protocol:

All the gel samples were prepared in demineralized water.

Amount = 70-80 mg of gel per HAP disc
Light source = Spectrum LED lamp 14 B 22
Distance between plate and lamp - 1.5 cm
Exposure time = 30 mins
Number of cycles: Four

Brushed with regular toothpaste after every cycle to removes excess gel A photograph to determine visual impact of the samples was taken and the and L*, a* and b* were measured after 4 cycles.

VISUAL data

Visual images of stained HAP disks after the treatment with samples of Examples 1 to 3 were observed followed by light exposure
Number of cycles = 4

Gel compositions containing Eosin polymers (Example 1) and free eosin (Example 2) showed photobleach efficacy. Gel samples of Example 1 showed superior photobleaching as compared to that of Example 2.

L a b data

The ΔL values of stained HAP disks after treatment with the above gels was measured and the data is summarised in the table below:

| Examples | Sample | ΔL |
|---|---|---|
| 1 | 0.5% polymer effective concentration of eosin 0.02% | 40 |
| 2 | 0.02% Eosin in gel | 34 |
| 3 | Control | -10 |

The data in the above table indicates superior photobleaching of HAP disks with the composition of the invention (Example -1) as compared to a composition where the dye is simply formulated in a gel.

Whitening Index observed (WIO) data

WIO values for the same samples were measured and the data is summarized in the table below:

| Examples | Sample | WIO |
|---|---|---|
| 1 | 0.5% polymer effective concentration of eosin 0.02% Eosin | 78 |
| 2 | 0.02% Eosin in gel | 65 |
| 3 | Control | -20 |

WIO measure confirms a trend similar to that obtained with ΔL measurement as well as visual observation where the polymeric dye was found to be superior to the other samples.

## Claims

1. A polymer having the structure:

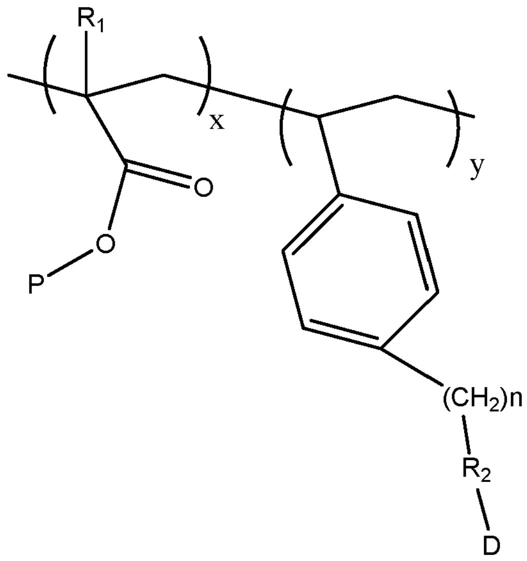

Where

$R_1$ is $C_n H_{2n+1}$
$R_2$ is a diheterofunctional group
n has a value from 1 to 10
x has a value from 1 to 10,000
y has a value from 1 to 10,000
P is a polyether chain; and
D is a Xanthene dye selected from at least one of Acid Red 52, Acid Red 289, Gallein, Bromopyrogallol Red, 2',7'-Dichlorofluorescein Sodium Salt, 9-(4'-Dimethylaminophenyl)-2,6,7-trihydroxyfluorone Sulfate Hydrate, Dibromofluorescein, Uranine K, Fluorescein, Uranine, Pyrogallol Red, Sulfonfluorescein, Tetrabromofluorescein Potassium Salt, Acid Red 91 ( Eosin Bluish), Acid Red 94 (Rose Bengal), Acid Red 87( Eosin), Acid Red 92 (Phloxine B ), 3,4,5,6-Tetrachlorofluorescein, Tetraiodofluorescein (Erythrosin B or Erythrosine B) and Phenylfluorone

2. A polymer as claimed in claim 1 wherein said $R_1$ is $CH_3$.

3. A polymer as claimed in claim 1 or 2 wherein said $R_2$ is an -O- group.

4. A polymer as claimed in any one of the preceding claims wherein x has a value from 5 to 100.

5. A polymer as claimed in any one of the preceding claims wherein y has a value from 5 to 100.

6. A polymer as claimed in any one of the preceding claims where P is a polyethylene glycol chain having the structure

P = Polyethylene glycol chain

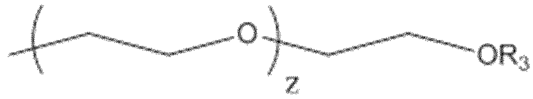

R3 = H or Phosphate or -CnH2n+1

And z has a value from 1 to 10.

7. A polymer as claimed in any one of the preceding claims wherein D is at least one of Rose Bengal, Eosin, Phloxine B, Fluorescien or Erythrosine.

8. A polymer as claimed in claim 7 wherein D is an eosin group having the chemical structure:

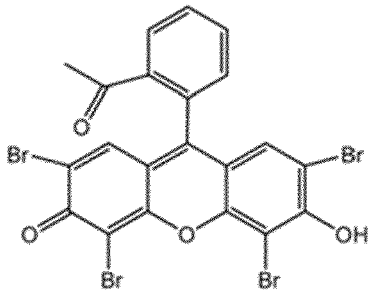

Eosin

9. An oral care composition comprising:

(i) a polymer as claimed in any one of the preceding claims; and
(ii) a cosmetically acceptable carrier.

10. A process to prepare a polymer as claimed in claim 1 comprising the steps of

(a) synthesizing the dye monomer by refluxing a mixture of the dye, chloromethylstyrene, potassium carbonate, and dimethyl formamide for six to ten hours; washing with salt solution, drying, filtering and concentrating to prepare the dye monomer; and
(b) synthesizing the dye polymer by repeated cycles of reacting OEGMA (oligo(ethylene glycol) methyl ether acrylate), dye monomer, AIBN (Azobisisobutyronitrile) and dioxane under an inert atmosphere for 1-10 hours at a temperature in the range of 20 to 70 °C, and precipitating in excess ether; followed by drying under vacuum at low temperature in the range of 20 to 40 °C.

11. A kit-of-parts comprising:

(i) A composition as claimed in claim 9;
(ii) a source of actinic/ Visible/LED light;
(iii) optionally, an applicator for applying said composition to teeth; and,
(iv) further optionally, a set of instructions for use of said kit-of-parts.

12. A method of whitening teeth comprising:

(i) Applying the composition as claimed in claim 9 on to a tooth surface; and
(ii) exposing said composition, so applied, to a source of actinic/ Visible/LED.

13. Use of a composition as claimed in claim 9 for whitening teeth.

**Patentansprüche**

1. Polymer mit der Struktur:

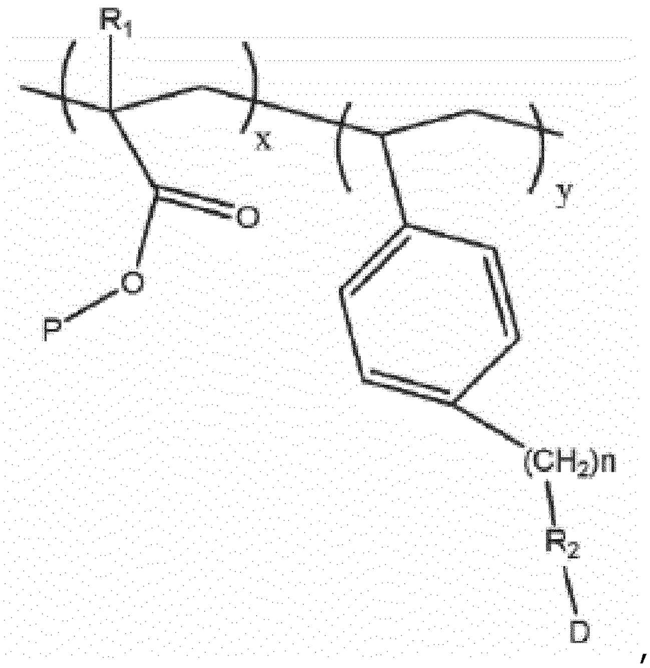

,

worin

$R_1$ $C_nH_{2n+1}$ ist,
$R_2$ eine diheterofunktionelle Gruppe ist,
n einen Wert von 1 bis 10 hat,
x einen Wert von 1 bis 10.000 hat,
y einen Wert von 1 bis 10.000 hat,
P eine Polyetherkette ist; und
D ein Xanthen-Farbstoff ist, ausgewählt aus mindestens einem von Acid Red 52, Acid Red 289, Gallein, Bromopyrogallol Red, 2',7'-Dichlorfluorescein-Natriumsalz, 9-(4'-Dimethylaminophenyl)-2,6,7-trihydroxyfluoron-Sulfathydrat, Dibromfluorescein, Uranine K, Fluorescein, Uranin, Pyrogallol Red, Sulfofluorescein, Tetrabromfluorescein-Kaliumsalz, Acid Red 91 (Eosin Bluish), Acid Red 94 (Rose Bengal), Acid Red 87 (Eosin), Acid Red 92 (Phloxin B), 3,4,5,6-Tetrachlorfluorescein, Tetraiodfluorescein (Erythrosin B oder Erythrosine B) und Phenylfluoron.

**2.** Polymer, wie im Anspruch 1 beansprucht, wobei das $R_1$ $CH_3$ ist.

**3.** Polymer, wie im Anspruch 1 oder 2 beansprucht, wobei $R_2$ eine -O-Gruppe ist.

**4.** Polymer, wie in irgendeinem der vorhergehenden Ansprüche beansprucht, wobei x einen Wert von 5 bis 100 aufweist.

**5.** Polymer, wie in irgendeinem der vorhergehenden Ansprüche beansprucht, wobei y einen Wert von 5 bis 100 aufweist.

**6.** Polymer, wie in irgendeinem der vorhergehenden Ansprüche beansprucht, wobei P eine Polyethylenglykol-Kette mit der folgenden Struktur
P = Polyethylenglykol-Kette

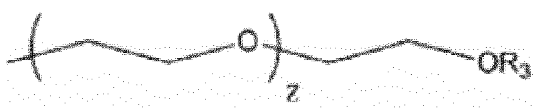

ist,
wobei

$R_3$ = H oder Phosphat oder $-C_nH_{2n+1}$ ist, und
z einen Wert von 1 bis 10 aufweist.

7. Polymer, wie in irgendeinem der vorhergehenden Ansprüche beansprucht, wobei D mindestens eines von Rose Bengal, Eosin, Phloxin B, Fluorescein oder Erythrosine ist.

8. Polymer, wie im Anspruch 7 beansprucht, wobei D eine Eosin-Gruppe der folgenden chemischen Struktur ist:

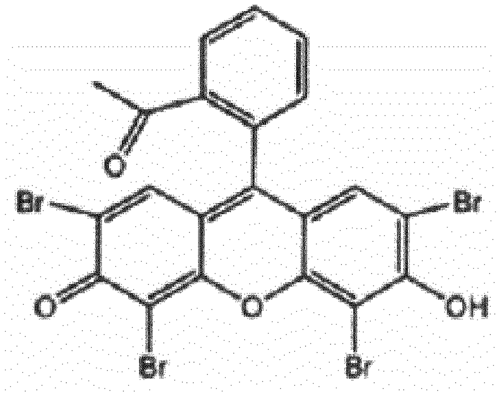

Eosin.

9. Mundpflegezusammensetzung, umfassend:

(i) ein Polymer, wie in irgendeinem der vorhergehenden Ansprüche beansprucht, und
(ii) einen kosmetisch akzeptablen Träger.

10. Verfahren zur Herstellung eines Polymers, wie im Anspruch 1 beansprucht, umfassend die Schritte:

(a) Synthetisieren des Farbstoffmonomers durch sechs- bis zehnstündigen Rückfluss einer Mischung von Farbstoff, Chlormethylstyrol, Kaliumcarbonat und Dimethylformamid, Waschen mit Salzlösung, Trocknen, Filtrieren und Konzentrieren zur Herstellung des Farbstoffmonomers; und
(b) Synthetisieren des Farbstoffpolymers durch wiederholte Zyklen der Umsetzung von OEGMA (Oligo(ethylenglycol) methyletheracrylat), Farbstoffmonomer, AIBN (Azobisisobutyronitil) und Dioxan unter inerter Atmosphäre für 1-10 Stunden bei einer Temperatur in dem Bereich von 20 bis 70°C und Ausfällen in überschüssigem Ether, gefolgt von Trocknen unter Vakuum bei niedriger Temperatur im Bereich von 20 bis 40°C.

11. Teilesatz, umfassend:

(i) eine Zusammensetzung, wie im Anspruch 9 beansprucht;
(ii) eine Quelle von aktinischem/sichtbarem/LED-Licht;
(iii) optional einen Applikator zum Auftragen der Zusammensetzung auf die Zähne; und
(iv) ferner optional einen Satz von Gebrauchsanweisungen für den Teilesatz.

12. Verfahren zum Aufhellen von Zähnen, umfassend:

(i) Auftragen der Zusammensetzung, wie im Anspruch 9 beansprucht, auf eine Zahnoberfläche; und
(ii) Bestrahlen der so aufgetragenen Zusammensetzung mit einer aktinischen/sichtbaren/LED-Quelle.

13. Verwendung einer Zusammensetzung, wie im Anspruch 9 beansprucht, zum Aufhellen von Zähnen.

## Revendications

1. Polymère ayant la structure

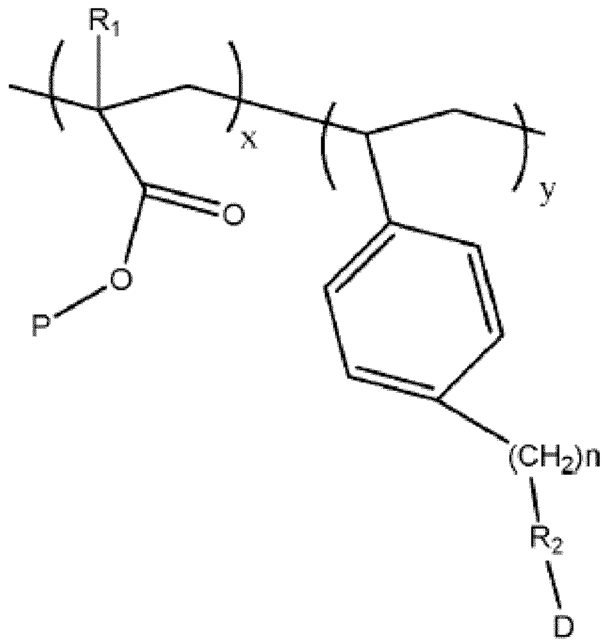

où

$R_1$ est $C_nH_{2n+1}$
$R_2$ est un groupe dihétérofonctionnel
n présente une valeur de 1 à 10
x présente une valeur de 1 à 10 000
y présente une valeur de 1 à 10 000
P est une chaîne polyéther ; et
D est un colorant de xanthène choisi parmi au moins un de rouge acide 52, rouge acide 289, galléine, rouge de bromopyrogallol, sel de 2',7'-dichlorofluorescéine sodium, hydrate de sulfate de 9-(4'-diméthylaminophényl)-2,6,7-trihydroxyfluorone, dibromofluorescéine, uranine K, fluorescéine, uranine, rouge de pyrogallol, sulfonfluorescéine, sel de tétrabromofluorescéine potassium, rouge acide 91 (éosine bleuâtre), rouge acide 94 (rose bengale), rouge acide 87 (éosine), rouge acide 92 (phloxine B), 3,4,5,6-tétrachlorofluorescéine, tétraiodo-fluorescéine (érythrosin B ou érythrosine B) et phénylfluorone.

2. Polymère selon la revendication 1, dans lequel ledit $R_1$ est $CH_3$.

3. Polymère selon la revendication 1 ou 2, dans lequel ledit $R_2$ est un groupe -O-.

4. Polymère selon l'une quelconque des revendications précédentes, dans lequel x présente une valeur de 5 à 100.

5. Polymère selon l'une quelconque des revendications précédentes, dans lequel y présente une valeur de 5 à 100.

6. Polymère selon l'une quelconque des revendications précédentes où P est une chaîne polyéthylène glycol ayant la structure

chaîne P-polyéthylène glycol

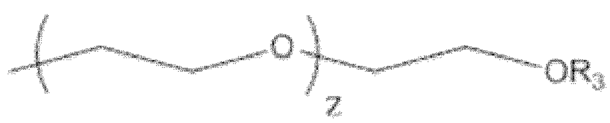

R3 = H ou phosphate ou -CnH2n+1

et z présente une valeur de 1 à 10.

**7.** Polymère selon l'une quelconque des revendications précédentes, dans lequel D est au moins un parmi rose bengale, éosine, phloxine B, fluorescéine ou érythrosine.

**8.** Polymère selon la revendication 7, dans lequel D est un groupe éosine ayant la structure chimique :

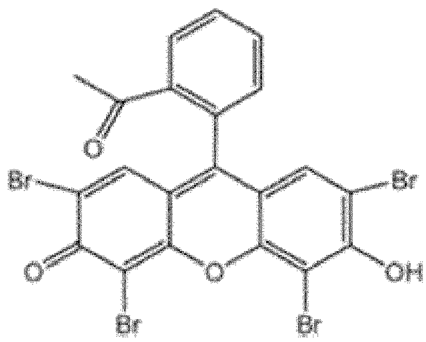

Eosine

**9.** Composition pour le soin oral comprenant :

(i) un polymère selon l'une quelconque des revendications précédentes ; et
(ii) un support cosmétiquement acceptable.

**10.** Procédé pour la préparation d'un polymère selon la revendication 1 comprenant les étapes de

(a) synthèse du monomère de colorant par reflux d'un mélange du colorant, de chlorométhylstyrène, carbonate de potassium, et diméthylformamide pendant de six à dix heures ; lavage avec une solution de sel, séchage, filtration et concentration pour préparer le monomère de colorant ; et
(b) synthèse du polymère de colorant par des cycles répétés de réaction de OEGMA (acrylate d'oligo(éthylène glycol)méthyléther), monomère de colorant, AIBN (azobisisobutyronitrile) et dioxane sous une atmosphère inerte pendant 1-10 heures à une température dans l'intervalle de 20 à 70°C, et précipitation dans de l'éther en excès ; suivie par séchage sous vide à basse température dans l'intervalle de 20 à 40°C.

**11.** Kit de pièces comprenant :

(i) une composition selon la revendication 9 ;
(ii) une source de lumière actinique/visible/LED ;
(iii) éventuellement, un applicateur pour appliquer ladite composition aux dents ; et,
(iv) de plus éventuellement, un jeu d'instructions pour une utilisation dudit kit de pièces.

**12.** Procédé de blanchiment des dents comprenant :

(i) l'application de la composition selon la revendication 9 sur une surface de dent ; et
(ii) l'exposition de ladite composition, ainsi appliquée, à une source actinique/visible/LED.

**13.** Utilisation d'une composition selon la revendication 9 pour le blanchiment de dents.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 2002122776 A
- WO 0018366 A1
- EP 1935395 A1
- US 2009238778 AA
- WO 2010149534 A
- US 20030194383 A

### Non-patent literature cited in the description

- **GARCIA JA ; RUBINO M ; ROMERO J ; HITA E.** Measuring The Whiteness Of Human Teeth. *Color Research and Application,* 1993, vol. 18, 349-52